# EUROPEAN PATENT SPECIFICATION

(11) **EP 0 440 199 B1**
(45) Date of publication and mention of the grant of the patent: **09.08.1995**
(21) Application number: 91101214.4
(22) Date of filing: 30.01.1991
(51) Int. Cl.: C08G 65/32

(54) **Process for preparing (per) fluoropolyethers of controlled molecular weight**
Verfahren zur Herstellung von (Per)-Fluorpolyethern eines kontrollierbaren Molekulargewichts
Procédé de préparation de (per)fluoropolyéthers de poids moléculaire contrôlé

(30) Priority: 31.01.1990 IT 1920190
(43) Date of publication of application: 07.08.1991
(73) Proprietor: AUSIMONT S.p.A., I-20121 Milano (IT)
(72) Inventor: Marchionni, Giuseppe, Dr., I-20133 Milan (IT); Gregorio, Guglielmo, Dr., I-20161 Milan (IT); De Patto, Ugo, Dr., I-20020 Cogliate, Milan (IT); Padovan, Mario, I-20125 Milan (IT)
(74) Representative: Barz, Peter, Dr.

(56) References cited:
- EP-A- 0 167 258
- EP-A- 0 223 238
- EP-A- 0 224 201

## Description

The present invention relates to the preparation of fluoropolyethers and perfluoropolyethers having neutral and/or functional end groups and exhibiting a lower controlled molecular weight than the corresponding higher molecular weight starting fluoropolyethers and perfluoropolyethers.

In particular, the present invention relates to an improved process for the selective catalytic cleavage of high molecular weight fluoropolyethers and perfluoropolyethers in order to obtain fluoropolyethers and perfluoropolyethers having neutral or functional end groups and exhibiting a lower and selectively modifiable molecular weight.

More specifically, the present invention relates to the catalytic cleavage of higher molecular weight fluoropolyethers and perfluoropolyethers obtained, for example, by photooxidation of perfluoroolefins, ring-opening polymerization of partially fluorinated oxetanes and/or fluorination of oxidized hydrogenated polyalkylene compounds, etc.

It is generally known that the methods employed for preparing the above (per)fluoropolyethers result in final products having, at least predominantly, too high a molecular weight (see, for example, GB-A-1,226,566 and 1,104,482 and US-A 3,250,808).

These high molecular weight products have limited practical use. In fact, among the most interesting applications of (per)fluoropolyethers are those in the electronic field, wherein very low molecular weight perfluoropolyethers are required, and in the field of operational fluids, for example for high-vacuum pumps, wherein also the use of medium molecular weight perfluoropolyethers is possible.

Thus, processes have been described for the catalytic degradation or cleavage of high molecular weight perfluoropolyethers in order to obtain perfluoropolyethers having a lower molecular weight.

EP-A-167,258 and 223,238 describe catalytic cleavage processes wherein certain perfluoropolyethers are catalytically cleaved by means of heat, resulting in products having a lower molecular weight and the same chain structure as the starting products in the form of mixtures of compounds with both neutral and acid end groups. Employed are fluorides and oxyfluorides of transition metals such as Ti, V, Cr, Mn, Fe, Co, Ni, Cu, Zr, Mo, Zn, or of Al, Sn, Sb or precursors thereof, such as bromides, chlorides, oxides (which are converted into the fluorinated catalytic species under the process conditions) and temperatures ranging from 150°C to 380°C.

The cleavage can occur at the C-O bond of the perfluoropolyethers, resulting in more homogeneous and stable products with high yields (EP-A-167,258); the cleavage can also take place at chemically more stable points of the chain, if more effective catalysts and high temperatures are used, i.e., by operating under more severe product distillation and recycle conditions (EP-A-223,238).

Nevertheless, the residence times in the reactor are generally such that the substrate can undergo undesirable fragmentations prior to its discharge from the reactor, which fragmentations are due, in particular, to the cleavage of the end groups, which give rise to gaseous products. Furthermore, the total specific productivity, i.e., the amount of product per reaction time unit and catalyst weight unit, and the catalyst life-time are not always satisfactory.

Thus, it is desirable to have available high-efficiency catalysts which allow to increase the reaction rate without having to prolong the contact times with the catalyst, thereby avoiding the main cause of the undesired decomposition reactions which lead to the formation of gaseous products.

Thus, it is an object of the present invention to provide an improved process for preparing fluoropolyethers and perfluoropolyethers having neutral and/or functional end groups and having a controlled molecular weight by catalytic cleavage of fluoropolyethers and perfluoropolyethers having a higher molecular weight.

Another object is that of providing perfluoro-2,5- and -2,6-dimethyl-1,4-dioxane (see GB-A-1,051,649) which have good solvent properties.

A further object is the provision of a catalytic cleavage process for fluoropolyethers and perfluoropolyethers having a high, selectively modifiable molecular weight (e.g. of from 1500 to 30000)in order to obtain lower molecular weight (e.g. of from 250 to 1000) products having a wider range of uses.

A further object is to provide a catalyst system endowed with a high specific productivity and a prolonged life-time.

These and still other objects, which will be more clearly apparent to those skilled in the art from the following description, are achieved, according to the present invention, by a process for producing fluoropolyethers and perfluoropolyethers having neutral and/or functional end groups and a lower molecular weight, by means of a selective catalytic cleavage of the corresponding higher molecular weight fluoropolyethers and perfluoropolyethers, said process being characterized in that said cleavage is effected by operating in the presence of a catalyst system comprising a combination of silicon dioxide with at least one oxide, fluoride and/or oxyfluoride of a metal selected from Al, Ti, V, Cr, Co, Fe, Ni, Mo, Zn, Cu, Cd, Mn, Sb, Sn, Zr, Sc, Y, lanthanides (La and homologues), Pb, Mg and W.

It has surprisingly been found that when operating under the conditions according to the present invention the catalyst system activity and life-time is higher than those obtainable when using the known fluorinated metal catalysts.

More specifically the catalyst system defined hereinbefore comprises high specific surface area materials based on silicon dioxide in combination with at least one oxide, fluoride or oxyfluoride of a metal selected from those listed above.

Thus, according to the present invention it is, for instance, possible to employ binary systems consisting of silicon dioxide and of only one other metal oxide, fluoride or oxyfluoride, or ternary or polymetal systems, in which the silicon dioxide is (chemically or physically) combined with more than one oxide, fluoride and/or oxyfluoride as defined above.

In the case of lanthanide oxides, fluorides and oxyfluorides, La and its homologues are considered to be included in the definition.

Whenever used in the present invention, the expression "silicon dioxide combined with oxides of the above metals" refers, in particular, to a mixed oxide of silicon and said metals, either binary or ternary or polyoxide, for example consisting of:
a) silicates of the above metals in the microcrystalline or amorphous form;
b) amorphous, chemically homogeneous mixtures, for example prepared by: 1) co-precipitation in the form of a gel and subsequent calcination, or 2) hydrolysis of mixtures of the corresponding metal alcoholates and subsequent drying, preferably followed by calcination, and
c) one or more metal oxides supported on SiO₂, preferably silica gel or amorphous silicas. Said supporting can be achieved by conventional techniques.

Very often said products are already available on the market and/or at least preparable, as mentioned before, according to known techniques; refer e.g. to: J. of Non-Crystalline Solids 82 (1986), 97-102; J. Cat. 35 (1974), 225-231; J. Cat. 105, (1987), 511-520; Applied Cat. 32, (1987), 315-326; Langmuir 5, (1987), 563-567.

In similar manner, whenever used in the present specification, the expression "silicon dioxide combined with the fluorides and oxyfluorides of the above metals" refers, in particular, to fluorides and oxyfluorides carried on silicon dioxide.

Substantially conventional techniques are utilizable for supporting the envisaged fluorides and oxyfluorides on the silicon dioxide.

In particular, in the case of fluorides or oxyfluorides of the above metals carried on SiO₂, it is possible to directly impregnate silica, for example a silica gel, with the aqueous solution of the fluoride or oxyfluoride of the selected metal or metals, or with the aqueous solution of a salt of said metals, which salt is capable of readily affording the fluoride by treatment with HF or salts thereof; drying and calcination are then carried out, for example, at a temperature ranging from 200 to 700°C.

Alternatively, if it is desired to prepare catalysts containing fluorides which are not obtainable from aqueous solutions, an anhydrous porous silica, for example consisting of silicalite or of a silica gel calcined at high temperature (for example at about 900 to 1000°C) is impregnated with an organic solution of a compound (for example a chloride or a bromide) which can be converted, after drying, into a fluoride or an oxyfluoride
a) directly in situ under the reaction conditions, or
b) by treatment with a fluorinating agent compatible with silica, such as F₂, hypofluorites, COF₂, etc., at temperatures ranging from about -50°C to about 100°C.

For example, a catalyst consisting of AlF₃ carried on silica gel may be prepared by dissolving hydrated alumina in aqueous HF, impregnating microporous silica with this solution, and subsequent drying. In similar manner an aqueous solution of (NH₄)₂ZrF₆ is used to impregnate the silica in the case of ZrF₄.

In the case of a catalyst consisting of supported yttrium, lanthanum or zirconium oxyfluoride, one may proceed as follows: silica gel is impregnated with an aqueous solution of lanthanum or yttrium nitrates or zirconyl nitrate, dried at about 160°C and then impregnated once again with an aqueous ammonium fluoride solution in amounts of from 2 to 3 fluoride ions per metal atom. After calcination at 600 to 700°C, a product wherein the metal is present predominantly in the form of an oxyfluoride may be obtained.

Tin fluoride on silica is, for instance, obtainable by impregnating silica gel, previously calcined at about 900°C, with SnBr₄ in the liquid form or dissolved in a volatile solvent. After evaporation of the solvent, the material is treated with gaseous CF₃OF, which acts as a mild fluorinating agent, exchanges the bromine and affords the supported tin fluoride.

Examples of catalyst systems which have proved to be particularly effective are the following ones: silica-alumina, also of the commercial type, silica-titanium oxide, aluminium silicates with zeolite structure and in the acidic form, silica-chromium oxide, chromium oxide on silca gel, silica-V₂O₅, AlF₃ on silica, LaOF and ZrOF₂ on silica.

In the scope of the definitions given before, the silica content, expressed as SiO₂, generally ranges from about 10 to about 95, and preferably from about 50 to about 90 weight percent. Furthermore, the specific surface area of the catalytic combination is usually ≧ 10 m²/g, and preferably ≧ 50 m²/g.

Preferably the cleavage reaction is conducted at temperatures ranging from about 150 to about 380°C, according to operative modes which are substantially conventional in the known cleavage reactions of this kind.

The catalytic combination is particularly suitably used in fine particle form, for example in the form of a fine powder of granules having average sizes below about 0.1 mm, thereby favoring the homogeneous dispersion of the catalyst system in the reaction mass.

The amount of catalytic combination to be employed can vary over a wide range, depending on the various process parameters such as temperature, contact time, employed high moleular weight perfluoropolyether, metal species contained in the mixed oxides and the like.

Nevertheless it is usually preferred to employ the catalyst combination in amounts ranging from about 0.1% to about 25% by weight, particularly from about 0.5% to about 10% by weight, based on the (per)fluoropolyether present.

The perfluoropolyethers and fluoropolyethers having perfluoroalkyl end groups and/or functional end groups, for the sake of simplicity, hereinafter referred to as functionalized (per)fluoropolyethers are well known in the art and are described, along with the methods for preparing them, in several articles, patents and patent applications, e.g., in GB-A 1,104,482, 1,226,566; US-A-3,242,218, 3,250,808, 3,665,041, 3,715,378, 3,847,978 and 4,532,039; EP-A-148,482, 151,877, 191,490 and 224,201; WO 87/00538 and WO 87/02992; IT-B-1,189,469 and the article by D. Sianesi "Polieteri perfluorurati: sinthesi e caratterizzazione di una nuova classe di fluidi inerti" ("Perfluorinated Polyethers: synthesis and characterization of a new class of inert fluids"), La Chimica e l'Industria, 50, February 1968, 206-214.

Many of these perfluoropolyethers are marketed, for example under the tradenames Fomblin®, Krytox® amd Demnum®.

The process of the present invention is applicable, in particular, to neutral and/or functionalized perfluoropolyethers and fluoropolyethers of the following types:
(I) 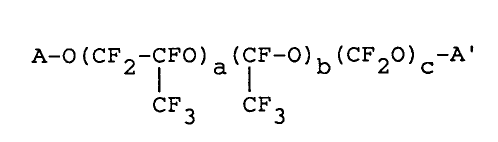 wherein A and A', the same or different from each other, represent a group selected from CF₃, C₂F₅, C₃F₇; COF, CF₂COCF₃,CF₂COF and CF(CF₃)COF;
   the units 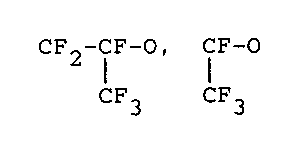 and CF₂O
   are statistically distributed along the chain;
   a and c are integers, while b is an integer or zero and the ratio a/b+c varies from 5 to 15; starting from such perfluoropolyethers, fractions of perfluoro-2,5-dimethyl-1,4-dioxane and/or perfluoro-2,6-dimethyl-1,4-dioxane may also be obtained.
(II)

   B-O-(C₂F₄O)_{d}(CF₂O)ₑ-B'

   wherein B and B', the same or different from each other, represent CF₃, C₂F₅, COF, CF₂-COF; the units C₂F₄O and CF₂O are statistically distributed along the chain; d and e are integers and the ratio d/e ranges from 0.3 to 5;
(III) 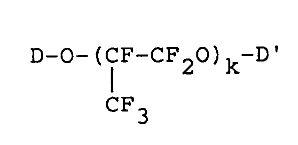 wherein D and D', the same or different from each other, represent C₃F₇ or 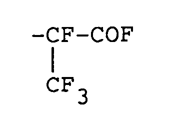 and k is an integer;
(IV) 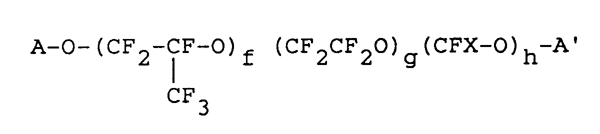 wherein A and A' are as defined above; X represents F or CF₃; the units in brackets are statistically distributed along the chain; f, g and h are integers; the ratio f/g+h ranges from 1 to 10 and the ratio g/h ranges from 1 to 10;
(V)

   E-O-(CF₂CF₂CF₂O)ⱼE'

   wherein E and E', the same or different from each other, represent CF₃, C₂F₅, C₃F₇, CF₂COF or CF₃CF₂COF and j represents an integer;
(VI) 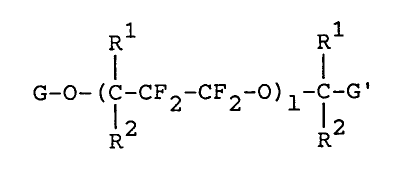 wherein G and G' are selected from 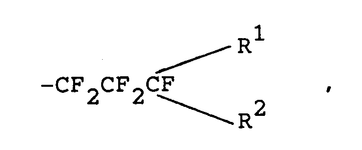 -CF₂-COF, -CF₂H, -CF₃ and derivatives of the fluoroacyl (COF) function, such as carboxy and salts thereof, amide, ester, etc.; R¹ and R², the same or different from each other, are selected from H, Cl, and optionally fluorinated alkyl radicals containing from 1 to 3 carbon atoms (e.g. CH₃ and C₂H₅); and l is an integer; when the compound contains different units of formula 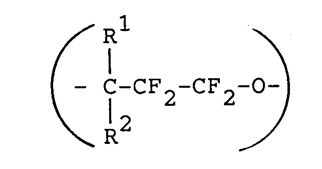 these units are statistically distributed along the chain;
(VII) 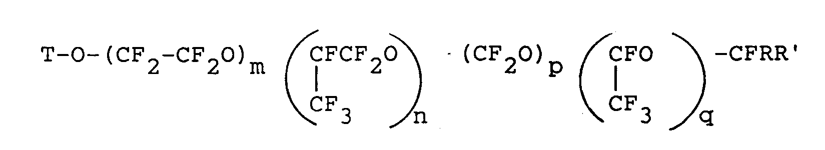 wherein T is a (preferably C₁-C₄-)perfluoroalkyl group containing a Cl atom, selected from, e.g.,
   CF₂Cl, CF₂ClCF₂, 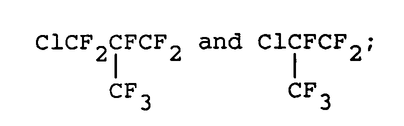 and R and R', the same or different from each other, represent fluorine or chlorine; the subscripts m, n, p, q are integers and can also be zero, provided that when m ranges from 1 to 20, n/m is equal to 0.02 to 2 and when n = 0 also q = 0.
   Said class (VII) is described in EP-A-340739.

According to preferred embodiments of the process of the present invention, a perfluoropolyether portion or a fluoropolyether portion is fed to a reactor connected with a distiller, the reactor being then heated to the desired temperature in the presence of the catalytic system employed according to the present invention. After a short induction period which can vary depending on, e.g., the temperature, the substrate to be cleaved and the particular type of catalyst system utilized but usually ranges from about 1 to about 30 minutes at a temperature of 280°C, the substrate cleavage begins, which is apparent from the formation of distillable liquids and gaseous by-products. Now, a gradual continuous feeding of the oil substrate begins, the level in the reactor being kept constant. At the same time, the cleavage products are distilled and the fraction corresponding to the non-cleaved and/or insufficiently cleaved starting oil is recycled.

A characteristic of the catalysts employed according to the present invention is that the amount of oil which can be processed before a decrease of the catalytic activity is observed is much higher than that obtained with conventional catalysts, e.g., up to 5 kg per gram of catalyst as compared to 0.4 kg per gram of catalyst with AlF₃ prepared by fluorination of alumina (prevailingly containing the gamma and delta forms).

Another characteristic is the specific productivity, which can reach levels of the order of 0.1 kg/g of catalyst/hour at 300°C, as compared to 0.02 kg/g of catalyst/hour obtained with AlF₃.

The exhausted catalysts, recovered at the end of the process, contain more or less considerable amounts of carbonaceous substances and the inorganic portion still contains silicon dioxide, although in lower amounts as compared to the starting silicon dioxide, the metal fluoride and/or oxyfluoride which had been fed. This material has a complex structure. A portion of the fluorides can be recognized as cystalline species, which is identifiable by the usual analytical techniques, while another portion remains in an amorphous form.

The present invention will now be described in more detail by reference to the following examples.

Examples 2, 4 and 12 were carried out, for comparative purposes, with the use of an AlF₃ catalyst according to the known technique, and evidence the lower yields, the lower productivity and the shorter life-time of the catalyst obtained thereby, as compared to the corresponding results obtained in the other examples which are in accordance with the present invention.

In the examples, PFPE means perfluoropolyether.

### EXAMPLE 1

Into a 0.1 l-glass reactor equipped with a glass distillation column of 20 cm length (⌀ = 2.5 cm) and packed with 5 mm Raschig rings, there were charged 80 g of PFPE of type (I) (A, A' = CF₃, C₂F₅, b = 0 and a/c = 10.9) having a viscosity of 251 cSt and an average molecular weight of about 3,600. To the PFPE charge, 7.5 g of SiO₂/Al₂O₃ (Akzo Chemie, having a content of 25% by weight of Al₂O₃ and a specific surface area of 525 m²/g) were added. The whole mass was then brought to a temperature of 278°C, whereafter the above PFPE was continuously fed at a flow rate of 318 g/h. The mixture of volatile products which was generated from the reaction mass passed through the column and the heavier products were recycled to the boiler. The head temperature at the top of the column was maintained at about 120 to 140°C. A distillate having an average molecular weight of 689, in an amount of 65.3% of the fed product, was obtained thereby. The distillation by-products were uncondensable gases essentially consisting of CO, CF₄ and C₂F₆. The above process was conducted continuously for 176 minutes without changing the operative conditions.

At the end of the process the catalyst was recovered by filtration and then washed. The catalyst was subjected to analysis, which revealed that the SiO₂ content was 30% of the starting content (X-ray fluorescence analysis). The analysis also revealed a fluorine content of 45% by weight and a specific surface area of 75 m²/g.

### EXAMPLE 2 (comparison)

In the apparatus of example 1, 15 g of the AlF₃ described in EP-A-167,258 were added to the PFPE charge as described in example 1. The whole mass was brought to a reaction temperature of 258°C whereafter a continuous feeding of PFPE at a flow rate of 387 g/h was started. The head temperature of the column was maintained at about 120 to 140°C. A distillate was obtained in an amount of 57.7% of the fed product; said distillate had an average molecular weight of 699. The process was conducted continuously for 147 minutes.

A comparison of example 1 and example 2 shows that, by operating under suitable conditions for obtaining products having the same characteristics, higher yields are obtained with the catalysts based on mixed oxides.

### EXAMPLE 3

The procedure of example 1 was followed, using the above apparatus. To about 80 g of PFPE there were added 2.3 g of SiO₂/Al₂O₃ of the type used in example 1.

After having reached a temperature of 271°C, the PFPE described in example 1 was continuously fed. By keeping the head temperature at about 90 to 110°C, the feed flow rate was 281 g/h. The distillate thus obtained had an average molecular weight of 608 and the yield was 64.8% based on the fed product. The process was conducted continuously for 30.4 hours. During this period of time the catalyst activity was nearly constant.

### EXAMPLE 4 (comparison)

Under the operative conditions described in example 3, there were utilized 2.3 g of AlF₃ of the type employed in example 2. After a temperature of 272°C had been reached, the same PFPE was fed continuously. By keeping a temperature of 100 to 110°C at the column head, the feed flow rate was 123 g/h. A distillate having an average molecular weight of 578 was obtained. In this case, the distillate yield was 53.2% referred to the fed product. The process was conducted continuously for 8.8 hours, whereafter the catalyst activity had rapidly decreased. A comparison of example 3 and example 4 shows that, although the corresponding distillates were very similar, in the case of the mixed SiO₂/Al₂O₃ oxide, higher yields, a higher productivity and a longer catalyst life-time were obtained.

### EXAMPLE 5

Into the apparatus used in example 1 there were charged 80 g of a PFPE of type (IV) (A, A' = CF₃, COF, f/g/h = 5.01/3.34/1.00) having a viscosity of 86 cSt and an average molecular weight of 2300, and 7.5 g of SiO₂/Al₂O₃ of the type employed in example 1. The whole mass was brought to a reaction temperature of 250°C, whereafter the above PFPE was continuously fed at a flow rate of 172 g/h. The temperature at the column head was maintained at about 90 to 100°C. Thereby a distillate having an average molecular weight of 540 was obtained. The distillate yield was 76.3% based on the fed product. The process was continuously conducted for 324 minutes.

### EXAMPLE 6

According to the modalities described in example 1, there were charged 80 g of PFPE of type (V), (E, E' = C₂F₅, C₃F₇) having a viscosity of 58 cSt and an average molecular weight of 2,700 and 10 g of SiO₂/Al₂O₃ of the type employed in example 1. The whole mass was brought to a reaction temperature of 300°C, whereafter the above PFPE was continuously fed at a flow rate of 128 g/h. The temperature at the column head was maintained at 120 to 140°C and the distillate was obtained in an amount of 88.2% referred to the fed product. Its average molecular weight was 540. The process was conducted for a total of 475 minutes.

### EXAMPLE 7

According to the modalities described in example 1, 80 g of PFPE of type (II) (B, B' = CF₃, COF, d/e = 0.63) having a viscosity of 567 cSt and an average molecular weight of 13,000 and 2.3 g of SiO₂/Al₂O₃ of the type described in example 1 were charged. The whole mass was heated to a reaction temperature of 230°C, whereafter the above PFPE was continuously fed at a flow rate of 324 g/h. The temperature at the column head was kept at about 40 to 50°C. A distillate was obtained with a yield of 32% referred to the fed product. The average molecular weight of the distillate was 380. The process was continuously conducted for a total of 171 minutes.

### EXAMPLE 8

Following the procedure of example 1, 80 g of PFPE of the type described in example 1 and 2.3 g of catalyst were charged. The catalyst was an acidic zeolite of type Y in the form of a fine powder with a specific surface area of 900 m²/g, prepared by calcination of LZ Y 62 LINDE in ammoniacal form, marketed by UNION CARBIDE (SiO₂/Al₂O₃ = 5 moles/mole). The whole mass was brought to a temperature of 274°C, whereafter the above PFPE was continuously fed at a flow rate of 119 g/h. The temperature at the column head was maintained at about 110 to 130°C. Finally, a distillate was obtained with a yield of 62.5% referred to the fed product. The distillate had an average molecular weight of 594.

The process was conducted continuously for a total of 380 minutes.

### EXAMPLE 9

Following the procedure described in example 1, 80 g of PFPE of type (II) (B, B' = CF₃, COF, d/e = 1.03) having a viscosity of 96.3 cSt and an average molecular weight of 7,000 and, subsequently, 2.3 g of Cr₂O₃ carried on SiO₂ (20% of Cr₂O₃) were charged. The whole mass was heated up to a reaction temperature of 254°C and the above PFPE was continuously fed at a flow rate of 112 g/h. The temperature at the column head was maintained at about 98 to 105°C. A distillate was obtained with a yield of 75.2% referred to the fed product; its average molecular weight was 584. The process was conducted continuously for a total of 215 minutes.

### EXAMPLE 10

Operating according to the modalities described in example 1, there were charged 80 g of PFPE of type (II) (B, B' = CF₃, C₂F₅, d/e = 0.82) having a viscosity of 200 cSt and an average molecular weight of 9,000 and, subsequently, 5 g of V₂O₅ carried on SiO₂ (10% of V₂O₅). The whole mass was heated to a reaction temperature of 281°C, whereafter the above PFPE was continuously fed. The temperature at the column head was maintained at about 140°C. Thereby a distillate was obtained with a yield of 81.2% referred to the fed product. The average moelcular weight thereof was 680. The process was carried out continuously for a total of 131 minutes and the feed flow rate was 241 g/h.

### EXAMPLE 11

A catalyst consisting of AlF₃ on silica gel was prepared by dissolving hydrated alumina in aqueous HF at 15% up to saturation and by impregnating with this freshly prepared solution an equal weight amount of commercial microporous silica (Akzo F7) having a specific surface area of 285 m²/g. The product was dried at 300°C, resulting in a catalyst which contained 38% of AlF₃.

172 g of a perfluoropolyether of type (I) (A, A' = CF₃, C₂F₅, b = 0 and a/c = 22) having a viscosity of 53.5 cSt and an average molecular weight of 1,800, were charged into a 250 ml flask, along with 2.0 g of the catalyst specified hereinabove; the flask was equipped with a magnetic stirrer, a distillation head and a cooler.

The mixture was gradually heated and at a temperature of 275°C the reaction product began to distill. The test was concluded when all of the starting material had reacted and the temperature had reached 290°C.

Finally, a distillate was recovered with a yield of 89% by weight based on the charged oil; the distillate had an average molecular weight of 783.

The catalyst, after recovering and washing, exhibited a specific surface area of 235 m²/g.

### EXAMPLE 12 (comparison)

174 g of the perfluoropolyether described in the preceding example were charged into a 250 ml flask, along with 2.0 g of AlF₃ as described in example 2. The flask was equipped with a magnetic stirrer, a distillation head and a cooler.

The mixture was gradually heated and, once a temperature of 280°C was reached, the reaction product began to distill. The test was concluded when all of the starting product had reacted and the temperature in the boiler had reached 310°C.

At the end of the reaction, a distillate having an average molecular weight of 966 was recovered in an amount of 79% referred to the charged oil.

### EXAMPLE 13

Example 3 was repeated. After a 10 hour reaction, 1820 g of product were obtained, which was treated with excess CaO to neutralize present acids. The reaction mixture was then steam distilled under atmospheric pressure. The distillate was separated from water, thus obtaining 974 g of product free of functional groups.

The neutral product was further rectified under atmospheric pressure and the fraction having a b.p. of 66 to 67°C was collected (234 g).

Gas-chromatography showed a 93% purity. NMR analysis showed this fraction to consist of a mixture of perfluoro-2,5-dimethyl-1,4-dioxane and perfluoro-2,6-dimethyl-1,4-dioxane.

## Claims

1. Process for preparing (per)fluoropolyethers having neutral and/or functional end groups by means of selective catalytic cleavage of the corresponding higher molecular weight (per)fluoropolyethers, wherein said cleavage is effected in the presence of a catalytic system comprising a combination of silicon dioxide with at least one oxide, fluoride and/or oxyfluoride of a metal selected from Al, Ti, V, Cr, Co, Fe, Ni, Mo, Zn, Cu, Cd, Mn, Sb, Sn, Zr, Sc, Y, lanthanides, Pb, Mg and W.

2. Process according to claim 1, wherein said combination is a mixed oxide consisting of (a) at least one microcrystalline or amorphous silicate of a metal selected from those given in claim 1; or (b) chemically homogeneous amorphous mixtures of SiO₂ and metal oxide; or (c) one or more metal oxides carried on silica.

3. Process according to claim 1, wherein said combination comprises at least one metal fluoride or metal oxyfluoride supported on SiO₂.

4. Process according to any one of claims 1 to 3, wherein said combination is selected from silica-alumina, silica-titanium oxide, aluminium silicates with zeolitic structure in the acidic form, silica-chromium oxide, chromium oxide on silica gel, silica-V₂O₅, AlF₃ on silica, LaOF and ZrOF₂ on silica.

5. Process according to any one of the preceding claims, wherein the silica content, expressed as SiO₂, ranges from 10% to 95% by weight, particularly from 50% to 90% by weight of the catalytic combination.

6. Process according to any one of the preceding claims, wherein the specific surface area of the catalytic combination is equal to or higher than 10 m²/g.

7. Process according to any one of the preceding claims, wherein said catalytic combination is employed in the form of a fine powder having an average particle size of less than 0.1 mm.

8. Process according to any one of the preceding claims, wherein said catalytic combination is employed in an amount of from 0.1% to 25% by weight, particularly from 0.5% to 10% by weight, based on the weight of the starting (per)fluoropolyether.

9. Process according to any one of the preceding claims, which is conducted at a temperature of from 150°C to 380°C.

10. Process for the preparation of perfluoro-2,5-dimethyl-1,4-dioxane and/or perfluoro-2,6-dimethyl-1,4-dioxane,
wherein a perfluoropolyether having the formula: 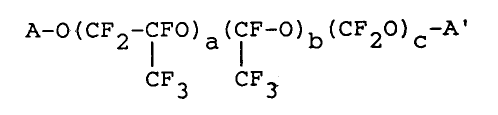 wherein A and A', the same or different from each other, represent a group selected from CF₃, C₂F₅, C₃F₇, COF, CF₂COCF₃, CF₂COF and CF(CF₃)COF;
the units in brackets are statistically distributed along the chain; a and c are integers, b is an integer or zero; and the ratio a/b+c ranges from 5 to 15;
is cleaved according to the process of any one of claims 1 to 9, the obtained mixture is distilled and the fraction boiling at 66-67°C is collected.

## Patentansprüche

1. Verfahren zur Herstellung von (Per)fluorpolyethern mit neutralen und/oder funktionellen Endgruppen mit Hilfe der selektiven katalytischen Spaltung der entsprechenden (Per)fluorpolyether mit höherem Molekulargewicht, in welchem die Spaltung in Anwesenheit eines katalytischen Systems bewirkt wird, welches eine Kombination von Siliciumdioxid mit mindestens einem Oxid, Fluorid und/oder Oxyfluorid eines Metalls umfaßt, das aus Al, Ti, V, Cr, Co, Fe, Ni, Mo, Zn, Cu, Cd, Mn, Sb, Sn, Zr, Sc, Y, den Lanthaniden, Pb, Mg und W ausgewählt ist.

2. Verfahren nach Anspruch 1, in welchem die Kombination ein gemischtes Oxid ist, das besteht aus (a) mindestens einem mikrokristallinen oder amorphen Silikat eines Metalls, das aus denjenigen, die in Anspruch 1 angegeben sind, ausgewählt ist; oder (b) chemisch homogenen amorphen Mischungen von SiO₂ und Metalloxid; oder (c) einem oder mehreren Metalloxiden, die sich auf einem Siliciumdioxid-Träger befinden.

3. Verfahren nach Anspruch 1, in welchem die Kombination mindestens ein Metallfluorid oder Metalloxyfluorid, das sich auf einem SiO₂-Träger befindet, umfaßt.

4. Verfahren nach irgendeinem der Ansprüche 1 bis 3, in welchem die Kombination ausgewählt ist aus Siliciumdioxid-Aluminiumoxid, Siliciumdioxid-Titanoxid, Aluminiumsilikaten mit Zeolith-Struktur in der sauren Form, Siliciumdioxid-Chromoxid, Chromoxid auf Kieselgel, Siliciumdioxid-V₂O₅, AlF₃ auf Siliciumdioxid, LaOF und ZrOF₂ auf Siliciumdioxid.

5. Verfahren nach irgendeinem der vorangehenden Ansprüche, in welchem der Siliciumdioxid-Gehalt, ausgedrückt als SiO₂, im Bereich von 10 bis 95 Gewichts-%, insbesondere 50 bis 90 Gewichts-%, der katalytischen Kombination liegt.

6. Verfahren nach irgendeinem der vorangehenden Ansprüche, in welchem die spezifische Oberfläche der katalytischen Kombination gleich oder höher als 10 m²/g ist.

7. Verfahren nach irgendeinem der vorangehenden Ansprüche, in welchem die katalytische Kombination in Form eines feinen Pulvers mit einer durchschnittlichen Teilchengröße von weniger als 0,1 mm eingesetzt wird.

8. Verfahren nach irgendeinem der vorangehenden Ansprüche, in welchem die katalytische Kombination in einer Menge von 0,1 bis 25 Gewichts-%, insbesondere 0,5 bis 10 Gewichts-%, bezogen auf das Gewicht des Ausgangs-(Per)fluorpolyethers, eingesetzt wird.

9. Verfahren nach irgendeinem der vorangehenden Ansprüche, das bei einer Temperatur von 150°C bis 380°C durchgeführt wird.

10. Verfahren zur Herstellung von Perfluor-2,5-dimethyl-1,4-dioxan und/oder Perfluor-2,6-dimethyl-1,4-dioxan, in welchem ein Perfluorpolyether mit der Formel: 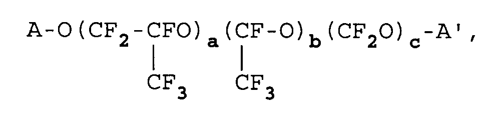 worin A und A', gleich oder verschieden voneinander, eine aus CF₃, C₂F₅, C₃F₇, COF, CF₂COCF₃, CF₂COF und CF(CF₃)COF ausgewählte Gruppe darstellen;
wobei die Einheiten in Klammern statistisch entlang der Kette verteilt sind; a und c ganze Zahlen sind, b eine ganze Zahl oder 0 ist; und das Verhältnis a/b+c im Bereich von 5 bis 15 liegt;
gemäß dem Verfahren von irgendeinem der Ansprüche 1 bis 9 gespaltet wird, die erhaltene Mischung destilliert wird und die bei 66 - 67°C siedende Fraktion gesammelt wird.

## Revendications

1. Procédé de préparation de (per)fluoropolyéthers possédant des groupes terminaux neutres et/ou fonctionnels par coupure catalytique sélective des (per)fluoropolyéthers correspondants de poids moléculaire plus élevé, dans lequel ladite coupure est effectuée en présence d'un système catalytique comprenant une combinaison de dioxyde de silicium avec au moins un oxyde, fluorure et/ou oxyfluorure d'un métal choisi parmi Al, Ti, V, Cr, Co, Fe, Ni, Mo, Zn, Cu, Cd, Mn, Sb, Sn, Zr, Sc, Y, lanthanides, Pb, Mg et W.

2. Procédé selon la revendication 1, dans lequel ladite combinaison est un oxyde mixte consistant en:
(a) au moins un silicate microcristallin ou amorphe d'un métal choisi parmi ceux de la liste donnée dans la revendication 1,; ou
(b) des mélanges amorphes chimiquement homogènes de SiO₂ et d'oxyde métallique; ou
(c) un ou plusieurs oxydes métalliques supportés sur de la silice.

3. Procédé selon la revendication 1, dans lequel ladite combinaison comprend au moins un fluorure métallique ou un oxyfluorure métallique supporté sur SiO₂.

4. Procédé selon l'une quelconque des revendications 1 à 3, dans lequel ladite combinaison est choisie parmi silice-alumine, silice-oxyde de titane, silicates d'aluminium à structure zéolitique sous forme acide, silice-oxyde de chrome, oxyde de chrome sur gel de silice, silice-V₂O₅, AlF₃ sur silice, LaOF et ZrOF₂ sur silice.

5. Procédé selon l'une quelconque des revendications 1 à 4, dans lequel la teneur en silice, exprimée en SiO₂, est comprise entre 10% et 95% en poids, en particulier entre 50 et 90% en poids par rapport à la combinaison catalytique.

6. Procédé selon l'une quelconque des revendications 1 à 5, dans lequel l'aire superficielle spécifique de la combinaison catalytique est égale ou supérieure a 10 m²/g.

7. Procédé selon l'une quelconque des revendications 1 à 6, dans lequel ladite combinaison catalytique est utilisée sous la forme d'une poudre fine présentant une dimension particulaire moyenne inférieure à 0,1 mm.

8. Procédé selon l'une quelconque des revendications 1 à 7, dans lequel ladite combinaison catalytique est employée à raison de 0,1 à 25% en poids, en particulier entre 0,5 à 10% en poids exprimés par rapport au poids du (per)fluoropolyéther de départ.

9. Procédé selon l'une quelconque des revendications 1 à 8, dans lequel on met en oeuvre le procédé à une température de 150°C à 380°C.

10. Procédé de préparation de perfluoro-2,5-diméthyl-1,4-dioxane et/ou perfluoro-2,6-diméthyl-1,4-dioxane, dans lequel un perfluoropolyéther répondant à la formule: 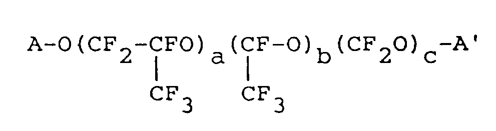 dans laquelle:
A et A', identiques ou différents, représentent un groupe choisi parmi CF₃, C₂F₅, C₃F₇, COF, CF₂COCF₃, CF₂COF, et CF(CF₃)COF;
les unités entre parenthéses sont statistiquement distribuées le long de la chaîne;
a et c sont des nombres entiers;
b est un nombre entier ou égal à zéro; et
le rapport a/b+c est compris entre 5 et 15;
est coupé par le procédé selon l'une quelconque des revendications 1 à 9, le mélange obtenu est distillé et la fraction bouillant à 66 à 67°C est recueillie.
